# EUROPÄISCHE PATENTSCHRIFT

(11) **EP 1 464 705 B1**
(45) Veröffentlichungstag und Bekanntmachung des Hinweises auf die Patenterteilung: **15.03.2006**
(21) Anmeldenummer: 04006966.8
(22) Anmeldetag: 01.12.1998
(51) Int. Cl.: C12N 15/12, C12N 15/65, C12N 15/67, C12N 15/85, C12N 15/90, C12N 5/10, C07K 14/505, C12Q 1/68

(54) **Optimierung von Zellen für die endogene Genaktivierung**
Optimizing cells for endogenous gene activation
Optimalisation de cellules pour l'activation de l'expression de gènes endogènes

(30) Priorität: 01.12.1997 EP 97121075
(43) Veröffentlichungstag der Anmeldung: 06.10.2004
(62) Teilanmeldung aus: 99112607.9
(73) Patentinhaber: Roche Diagnostics GmbH, 68305 Mannheim (DE)
(72) Erfinder: Honold, Konrad, 82377 Penzberg (DE); Holtschke, Thomas, 81369 München (DE); Stern, Anne, 82377 Penzberg (DE)
(74) Vertreter: Weiss, Wolfgang

(56) Entgegenhaltungen:
- EP-A- 0 343 783
- EP-A- 0 747 485
- WO-A-91/09955
- WO-A-94/17176
- WO-A-96/29411
- WO-A-96/30498
- US-A- 4 959 317
- DATABASE WPI Section Ch, Week 198801 Derwent Publications Ltd., London, GB; Class B04, AN 1988-003020 XP002118545 & JP 62 265992 A (AJINOMOTO KK) 18. November 1987 (1987-11-18)
- FUKUSHIGE S ET AL: "GENOMIC TARGETING WITH A POSITIVE-SELECTION LOX INTEGRATION VECTOR ALLOWS HIGHLY REPRODUCIBLE GENE EXPRESSION IN MAMMALIAN CELLS" PROCEEDINGS OF THE NATIONAL ACADEMY OF SCIENCES OF USA, NATIONAL ACADEMY OF SCIENCE. WASHINGTON, US, Bd. 89, Nr. 17, 1. September 1992 (1992-09-01), Seiten 7905-7909, XP000615553 ISSN: 0027-8424
- WANG P ET AL: "HIGH FREQUENCY RECOMBINATION BETWEEN LOXP SITES IN HUMAN CHROMOSOMES MEDIATED BY AN ADENOVIRUS VECTOR EXPRESSING CRE RECOMBINASE" SOMATIC CELL AND MOLECULAR GENETICS, NEW YORK, NY, US, Bd. 21, Nr. 6, 1. November 1995 (1995-11-01), Seiten 429-441, XP000617918

## Beschreibung

Die Erfindung betrifft Verfahren zur Optimierung der Genexpression in Zellen. Die Erfindung betrifft ein Verfahren zur Bereitstellung einer eine Rekombinase-Zielsequenz enthaltende DHFR-negative eukaryontische Zelle sowie die Expression einer in der Rekombinase-Zielsequenz inserierten Nukleinsäuresequenz.

Die Genexpression in einer Zelle kann konstitutiv, beispielsweise bei sogenannten Housekeeping-Genen, oder reguliert erfolgen. Die regulierte Expression ist insbesondere für Gene notwendig, die nur in einem bestimmten Entwicklungsstadium der Zelle oder bei einer Änderung der Umweltbedingungen exprimiert werden müssen.

Die Expression wird auf der Transkriptionsebene durch den operativ mit der kodierenden Nukleinsäuresequenz verbundenen Promotor reguliert, dessen

Aktivität durch Repressoren und Aktivatoren gesteuert werden kann. Eine Bindung von Repressoren bzw. Aktivatoren an nichtkodierende Nukleinsäuresequenzen des Gens kann eine Verminderung bzw. Erhöhung der Aktiviät des Promotors bewirken (L. Stryer, Biochemie, Kapitel 12, Spektrum der Wissenschaft, Verlagsgesellschaft, Heidelberg, 1990). Die Menge der in einer Zelle enthaltenen Repressoren bzw. Aktivatoren wird wiederum durch Faktoren, wie beispielsweise Umweltbedingungen, reguliert. Ein Beispiel für Aktivatoren sind die Hypoxia-Inducible-Factoren (HIF), die durch vermindertes O₂-Angebot induziert werden und zu einer erhöhten Expression des Erythropoietingens führt (Blanchard K.L. et al., Hypoxic induction of the human erythropoietin gene: Cooperation between the promotor and enhancer, each of which contains steroid receptor response elements, (1992), Mol.Cell.Biol. 12, 5373-5385; Wang G.L. and Semenza G.L., Characterization of hypoxia-inducible factor 1 and regulation of DNA binding activity by hypoxia, (1993), J.Biol.Chem., 268, 21513-21518; Wang G.L. et al., Hypoxia-inducible factor 1 is a basic-helix-loophelix-PAS heterodimer regulated by cellular O₂ tension, (1995), Proc.Natl.Acad.Sci. USA, 92, 5510-5514).

Desweiteren ist die Menge eines exprimierten Proteins von der Stabilität der mRNA abhängig. Im 3'-seitigen Bereich einer mRNA sind Erkennungssequenzen für mRNA abbauende Enzyme lokalisiert, die die Stabilität der mRNA und somit die Expressionshöhe beeinflussen (Shaw G. and Kamen R., A Conserved AU Sequence from the 3'Untranslated Region of GM-CSF mRNA Mediates Selective mRNA Degradation, Cell (1986), 659-667). Die Halbwertszeit der mRNA korreliert dabei mit der Menge exprimierten Proteins. Eine dritte Ebene der Expressionsregulation ist die Translation.

Die Expression eines Gens unterliegt somit komplexen Regulationsmechanismen, die im Einzelfall sehr unterschiedlich sein können.

Proteine können mit Hilfe der rekombinanten DNA-Technologie gewonnen werden, welche die Kenntnisse der Expressionsregulation nutzt (Sambrook et al., 1989 Molecular Cloning, A Laboratory Manual, Cold Spring Harbor). Hierzu werden Vektoren verwendet, die eine das entsprechende Protein kodierende Nukleinsäuresequenz unter Kontrolle eines geeigneten Promotors enthalten, sowie weitere für die Expression des Proteins und zur Replikation des Vektors notwendige Sequenzen. Der Vektor wird dann mittels bekannter Verfahren in eine Wirtszelle eingebracht, die Zelle wird kultiviert und das rekombinante Protein kann aus der Zelle oder dem Kulturmedium gewonnen werden.

Als Wirtszelle können prokaryontische oder eukaryontische Zellen verwendet werden. Prokaryontische Zellen, insbesondere E.coli-Zellen, sind in ihrer Handhabung unproblematisch, weisen aber bei einer rekombinanten Expression von eukaryontischen Proteinen eine Reihe von Nachteilen auf.

Prokaryonten und Eukaryonten unterscheiden sich im Expressionsprozessierungsweg, in den Zellmilieu-Bedingungen sowie in den bei der Proteinprozessierung beteiligten Chaperons. Deshalb können in einem in Prokaryonten hergestellten eukaryontischen Protein entscheidende Unterschiede im Vergleich zu dem entsprechenden nativen Protein auftreten. Beispielsweise kann das Proteinfaltungsmuster und die Aktivität des Proteins verändert sein. Auch werden Proteine in einer prokaryontischen Wirtszelle in der Regel nicht glycosyliert. Ein korrektes Glycosylierungsmuster stellt aber in vielen Fällen, beispielsweise bei der Herstellung von Proteinen für eine pharmazeutische Formulierung, ein entscheidendes Merkmal für die Wirksamkeit und Verträglichkeit dar.

Glycosylierte Proteine werden deshalb mittels eukaryontischer Wirtszellen oder Zellinien, beispielsweise CHO (Chinese Hamster Ovary) Zellen, hergestellt. Trotz der Verwendung eukaryontischer Zellen können aufgrund von Speziesunterschieden, beispielsweise bei der Expression eines humanen Proteines in nichthumanen Zellen, Veränderungen in dem rekombinant hergestellten Protein auftreten, wodurch dieses für viele Anwendungen unbrauchbar wird.

Zur rekombinanten Herstellung von Proteinen werden Wirtszellen transient oder stabil mit Expressionsvektoren transfiziert, wobei insbesondere bei großtechnischen Herstellungsverfahren stabil transfizierte Zellen verwendet werden.

Die unspezifische, zufällige Integration der Expressionsvektorsequenzen in das Genom der Wirtszelle kann zu Zellen mit geringer Produktionsleistung oder instabilen Eigenschaften der Zellen führen. Beispielsweise kann im Laufe des Produktionsprozesses die Produktionsleistung sinken oder die Fähigkeit der Zellen das rekombinante Protein zu exprimieren geht ganz verloren.

Ein Verfahren zur Erhöhung der Genexpression stellt die Genamplifikation dar, bei der eine für ein Protein kodierende Nukleinsäuresequenz mit einem Amplifikationsgen gekoppelt wird. Durch einen Selektionsschritt erreicht man eine Vervielfältigung beider Sequenzen, die zu einer erhöhten Expression führt (Schimke R.T. (Ed.) (1982), Gene amplifikation, Cold Spring Harbor Lab., Cold Spring Harbor, NY).

EP 0 747 485 beschreibt ein Verfahren zur Herstellung von Säugetierproteinen durch Transformation von Säugetierwirtszellen mittels homologer Rekombination. Die Transformation der Zellen, welche ein endogenes Zielgen beinhalten, erfolgt mit Hilfe eines Vektors, der ein Amplifikationsgen und/oder eine heterologe Nukleotid-Kontrolisequenz sowie zumindest einen flankierenden Bereich umfasst, der zu einem Bereich des Wirtszellengenoms innerhalb des endogenen Zielgens homolog ist.

Als Amplifikationsgen kann beispielsweise eine für eine Dihydrofolatreduktase (DHFR) kodierende Nukleinsäure verwendet werden (Kaufmann R.J., Sharp P.A. (1982), Amplifikation and expression of sequences cotransfected with a modular dihydrofolate reductase complementary DNA gene, J. Mol. Biol. 159:601ff).

Durch einen mit Methotrexat durchgeführten Selektionsschritt erhält man Zellen, die gegenüber Methotrexat resistent sind und in ihrem Genom die für eine DHFRkodierende und mit ihr gekoppelte Nukleinsäuresequenz in 20 bis 50-facher Amplifikation enthalten (R. Knippers, 1982, Molekulare Genetik, Thieme, Stuttgart).

Ein derartiges Genamplifikationsverfahren wird am effektivsten mit einer DHFR-negativen Zelle durchgeführt. JP-62265992 beschreibt z.B. eine humane DHFR-negative Zelle. Ein Hinweis auf eine ortsspezifische Integration eines Expressionsvektors mittels homologer Rekombination und Amplifikation dieser Sequenzen in dieser Zelle findet sich darin jedoch nicht.

Auch bei der Durchführung eines Genamplifikationsverfahrens können aufgrund zufälliger Integration des Expressionsvektors in das Genom der Zelle die oben dargestellten Nachteile, wie beispielsweise Instabilität der Zellen, auftreten.

Lediglich bei einer ortsspezifischen Integration von Fremd-DNA an einem ausgewählten Genlocus durch homologe Rekombination, die zu einer endogenen Genaktivierung führt, können die beschriebenen Nachteile vermieden werden. Entsprechende Verfahren sind bekannt und werden als Gentargeting bezeichnet (WO 90/11354; WO 91/09955). Dabei wird die Zelle mit einem Vektor transfiziert, der ein positives Selektionsmarkergen enthält, flankiert von Nukleinsäuresequenzen, die homolog zu Sequenzen eines Genlocus sind, an dem der Vektor in das Genom der Zelle integriert werden soll. Zwischen den homologen Nukleinsäuresequenzen befindet sich weiterhin eine heterologe Expressionskontrollsequenz, um die Expression des Zielgens in der Zelle zu erhöhen, und gegebenenfalls ein Amplifikationsgen, um die Kopienzahl des Zielgens zu vergrößern.

Ein Nachteil bisher bekannter Gentargeting-Verfahren besteht darin, daß die Herstellung von Zellen mit Eigenschaften, die die Herstellung eines gewünschten Proteins in einer für kommerzielle Zwecke ausreichenden Menge und Qualität ermöglichen, oft mit sehr hohem Aufwand verbunden ist. Insbesondere die Auswahl von optimalen Expressionskontrollsequenzen oder/und Amplifikationsgenen für die Expression eines gewünschten Zielproteins erfordert oft eine beträchtliche Anzahl von Versuchsreihen zur homologen Rekombination, die aufgrund der aufwendigen Prozedur zur Isolierung von Klonen, in denen das gewünschte Rekombinationsereignis stattgefunden hat, mit sehr hohem Aufwand verbunden sind.

Die homologe Rekombination kann auch verwendet werden, um die Expression bestimmter Gene in einer Zelle auszuschalten und Protein-Funktionsstudien durchzuführen. Hierzu werden Knockout-Mäuse erzeugt, indem das für ein zu untersuchendes Protein kodierendes Gen in embryonalen Stammzellen durch homologe Rekombination ausgeschaltet wird. Nach Durchführung weiterer Verfahrensschritte werden Mäuse erhalten, die aufgrund der Inaktivierung beider Allele dieses Gens vom Beginn ihrer Entwicklung kein funktionelles Protein exprimieren können (Thomas K.R., Capecchi M.R., (1987), Site-directed mutagenesis by gene targeting in mouse embryo-derived stem cells, Cell 51: 503-512).

Um ein bestimmtes Gen gewebe- und zeitspezifisch auszuschalten und zu untersuchen, kann das Cre-Lox-System eingesetzt werden. Hierbei wird ein von zwei loxP-Sequenzen flankiertes Nukleinsäurefragment durch homologe Rekombination in das Genom einer Zelle eingebracht und kann anschließend durch eine in der Zelle exprimierte Cre-Rekombinase wieder aus dem Genom herausgeschnitten werden (Sauer B, Henderson N (1989): Site-specific DNA recombination at loxP sites placed into the genome of mammalian cells. Nuc Acid Res 17:147-161; Sauer B., Henderson N. (1990), Targeted insertion of exogenous DNA into the eukaryotic genome by the Cre recombinase, New Biol. 5:441-449).

WO 96/30498 beschreibt ein Verfahren zur Herstellung von Zellen, welche durch Modifikation des Genlocus zellspezifischer Immunglobuline mittels Cregesteuerter ortsspezifischer Rekombination einen modifizierten Antikörper exprimieren. Dies wird durch Transfektion der Zellen mit drei verschiedenen Vektoren realisiert, welche die Einführung zweier loxP-Regionen, einer modifizierenden Nukleotidsequenz und eines Selektionsmarkergens in den Immunglobulinlocus der Antikörper-produzierenden Zellen bewirken.

Die der vorliegenden Erfindung zugrunde liegende Aufgabe bestand darin, ein neues Verfahren zur Optimierung der endogenen Genaktivierung durch homologe Rekombination bereitszustellen, bei dem die Nachteile des Standes der Technik zumindest teilweise beseitigt werden.

Die Erfindung betrifft ein Verfahren zur Bereitstellung einer DHFR-negativen eukaryontischen Zelle, vorzugsweise einer Säugerzelle und besonders bevorzugt einer humanen Zelle, das dadurch gekennzeichnet ist, daß
(a) die Zelle transfiziert wird mit einem ersten Vektor, umfassend
   (i) mindestens eine Zielsequenz für eine ortsspezifische Rekombinase,
   (ii) die Sequenz (i) flankierende DNA-Sequenzen, die homolog zu einer endogen in der Zelle vorliegenden DHFR-Nukleinsäuresequenz sind, um eine homologe Rekombination zu erlauben, und
   (iii) gegebenenfalls ein positives und gegebenenfalls ein negatives Selektionsmarkergen,
(b) die transfizierte Zelle unter Bedingungen kultiviert wird, unter denen eine homologe Rekombination des Vektors erfolgt, und
(c) die gemäß Schritt (b) erhaltene Zelle gewonnen wird.

Das positive Selektionsmarkergen wird - sofern vorhanden - zwischen den zu einem DHFR-Gen homologen Sequenzen angeordnet. Das negative Selektionsmarkergen wird - sofern vorhanden - außerhalb der homologen Sequenzen angeordnet.

Die Bezeichnung "ortsspezifische Rekombinase" gemäß vorliegender Erfindung umfaßt Proteine und Proteinkomplexe, die DNA-Umlagerungen an einer spezifischen DNA-Zielsequenz vermitteln, einschließlich ortsspezifischer Rekombinasen der Integrase- oder Resolvase-Invertase-Klassen (Stark et al., Trends Genet. 8 (1992), 432-439; Abremski und Hoess, Protein Engineering 5 (1992), 87-91; Khan et al., Nucleic Acids Res. 19 (1991), 851-860) und durch Intron-kodierte Endonukleasen vermittelte ortsspezifische Rekombination (Perrin et al., EMBO J. 12 (1993), 2939-2947). Bevorzugte Rekombinaseproteine werden ausgewählt aus der Gruppe bestehend aus der FLP-Rekombinase des 2 *µ* Episoms von Saccharomyces cerevisiae (z.B. Falco et al., Cell 29 (1982), 573-584; Cox, Proc. Natl. Acad. Sci. USA 80 (1983) 4223-4227; Konsolaki et al., New Biologist 4 (1992), 551-557), der Cre-Rekombinase des E.coli Phagen P1 (z.B. Sauer und Henderson (1989) supra), der R-Rekombinase aus dem Zygosaccharomyces rouxii Plasmid pSR1 (Matsuzaki et al., J. Bacteriol. 172 (1990), 610-618), der A-Rekombinase aus dem Kluyveromyces drososphilarium Plasmid pKD1 (Chen et al., Nucleic Acids Res. 14 (1986), 4471-4481), der A-Rekombinase aus dem Kluveromyces waltii-Plasmid pKW1 (Chen et al., J. Gen. Microbiol. 138 (1992), 337-345), einer Komponente des λ-Int-Rekombinationssystems (Landy, Annu Rev. Biochem. 5( (1989), 913-949) und einer Komponente des Gin-Rekombinationssystems des Phagen *µ* (Klippel et al., EMBO J. 12 (1993), 1047-1057). Darüber hinaus sind auch die im europäischen Patent EP-B-O 707 599 beschriebenen Fusionsproteine aus einer ortsspezifischen Rekombinase und einem nuklearen Rezeptor oder der ligandenbindenden Domäne davon geeignet. Besonders bevorzugt werden für das erfindungsgemäße Verfahren Zielsequenzen der Cre-Rekombinase, d.h. loxP-Sequenzen, verwendet.

Die Auswahl geeigneter homologer Sequenzen erfolgt beispielsweise gemäß den in W090/11354 und WO91/09955 beschriebenen Methoden.

Darüber hinaus können in den homologen Sequenzen auch Modifikationen enthalten sein, die im exprimierten Protein zu Mutationen, wie beispielsweise Punktmutationen, Insertionen oder/und Deletionen einzelner Aminosäuren oder ganzer Aminosäureabschnitte führen.

Erfindungsgemäß kann jede eurkaryontische Zelle verwendet werden, vorzugsweise wird eine Säugerzelle, besonders bevorzugt eine humane Zelle verwendet. Das erfindungsgemäße Verfahren kann mit nichtimmortalisierten Zellen, z.B. Fibroblasten, aber auch mit immortalisierten Zellen, z.B. Tumorzellinien, durchgeführt werden. Bevorzugt sind immortalisierte Zellen.

Die bei der Durchführung des erfindungsgemäßen Verfahrens verwendeten Lösungen und Medien werden vorzugsweise so ausgewählt, daß im jeweiligen Verfahrensschritt optimale Bedingungen vorliegen. Die Kultivierung der Zellen erfolgt mit Medien, die alle für ein ausreichendes Zellwachstum nötigen Stoffe enthalten und gegebenenfalls gepuffert sind. Vorzugsweise sind die Zellen in serumfreiem Medium kultivierbar. Besonders bevorzugt ist die verwendete Zelle eine Namalwa-, HT1080 oder HeLa S3 Zelle.

Das Amplifikationsgen wird vorzugsweise in einer exprimierbaren Form, d.h. in operativer Verknüpfung mit einem geeigneten Promotor eingesetzt und im Vektor so angeordnet, daß es sich nach der homologen Integration des Vektors in das Genom der eukaryontischen Zelle in räumlicher Nähe zum Zielgen befindet. Die Durchführung eines Amplifikationsschrittes führt zu einer Erhöhung der Anzahl von Kopien des Zielgens in der Zelle. Hierdurch kann eine weitere Expressionssteigerung der endogenen Nukleinsäuresequenz erreicht werden. Beispiele für geeignete Amplifikationsgene sind Dihydrofolatreduktase (DHFR), Adenosindeaminase, Ornithindecarboxylase bzw. Muteine dieser Gene. Vorzugsweise ist das Amplifikationsgen ein DHFR-Gen oder eine mutierte Form davon (Simonsen et al., Nucleic Acids Res. 1988, 16 (5): 2235-2246), insbesondere bei Zellen, die ein endogenes DHFR-Gen enthalten.

Als positiver Selektionsmarker kann jedes für eine eukaryontische Zelle geeignete Resistenzgen verwendet werden, welches zu einem selektierbaren Phänotyp führt, wie z.B. eine Antibiotikumresistenz. Vorzugsweise ist das positive Selektionsmarkergen ein Neomycin-, Kanamycin-, Geneticin- oder Hygromycin-Resistenzgen. Vorzugsweise wird das positive Selektionsmarkergen in exprimierbarer Form, d.h. in operativer Verknüpfung mit einem geeigneten Promotor verwendet.

Wird ein negatives Selektionsmarkergen verwendet, so wird üblicherweise zusätzlich zu dem positiven Selektionsschritt ein zweiter negativer Selektionsschritt durchgeführt. Dies bietet den Vorteil, daß nach Durchführung der Selektionsschritte die identifizierten Klone einen geringeren Anteil falschpositiver Klone, d.h. zufällig in das Genom integrierte Vektoren, enthalten. Das negative Selektionsmarkergen ist vorzugsweise ein Thymidin-Kinase-Gen (TK) oder/und ein Hypoxanthin-Guanin-Phosphoribosyltransferase-Gen (HGPRT).

Alle erfindungsgemäßen Vektoren enthalten weiterhin vorzugsweise die für eine Propagierung und Vermehrung in geeigneten Wirtszellen nötigen Sequenzelemente, wie beispielsweise Replikationsursprung, Selektionsmarkergene etc.

Nach erfolgter homologer Rekombination in den DHFR-Locus kann kein funktionelles DHFR-Protein von der Zelle synthetisiert werden. Die Sequenzen des Vektors können dabei so angeordnet sein, daß der Promotor des DHFR-Gens inaktiviert wird oder/und daß aufgrund einer Insertion oder Deletion in der kodierenden Sequenz des DHFR-Gens kein funktionelles DHFR-Protein mehr synthetisiert werden kann.

Um beide Allele eines DHFR-Gens zu inaktivieren, werden die Zellen zunächst mit einem erfindungsgemäßen Vektor transfiziert, selektioniert und gewonnen. In diesen Zellen ist ein Allel des DHFR-Gens inaktiviert, d.h. sie sind heterozygot (+/-) für das DHFR-Gen. Dann können diese Zellen nochmals mit einem erfindungsgemäßen Vektor transfiziert werden, der vorzugsweise ein von dem ersten Vektor verschiedenes positives Selektionsmarkergen enthält. Nach einem Selektionsschritt werden Zellen gewonnen, in denen beide DHFR-Allele inaktiviert sind. Alternativ kann eine Erhöhung des Selektionsdrucks zu einer Genkonversion und damit zur Inaktivierung beider Allele führen (vgl. z.B. Mortensen et al., Mol. Cell. Biol. 12 (1992), 2391-2395).

Das erfindungsgemäße Verfahren stellt eine DHFR-negative Zelle bereit, deren Verwendung in einem Genamplifikationsverfahren den Vorteil hat, daß sie kein endogenes DHFR-Protein synthetisiert. Bei der Durchführung eines Selektionsschrittes zur Amplifikation einer heterologen Nukleinsäuresequenz, die mit einer für ein DHFR-Protein kodierenden Nukleinsäuresequenz gekoppelt ist, kommt es zu keinen störenden Einflüssen des Expressionsproduktes des endogenen DHFR-Gens und somit zu einer Effizienzsteigerung der Genamplifikation.

Als positives Selektionsmarkergen kann jedes geeignete Selektionsmarkergen verwendet werden, welches zu einem selektierbaren Phänotyp führt, z.B. Antibiotikumresistenz. Vorzugsweise ist die für das positive Selektionsmarkergen kodierende Nukleinsäuresequenz ein Neomycin-, Kanamycin-, Geneticin- oder Hygromycin-Resistenzgen.

Es kann jedes dem Fachmann bekannte negative Selektionsmarkergen verwendet werden, vorzugsweise ist die für das negative Selektionsmarkergen kodierende Nukleinsäuresequenz ein Thymidin-Kinase-Gen (TK) oder/und Hypoxanthin-Guanin-Phosphoribosyltransferase-Gen (HGPRT).

Die von Rekombinase-Zielsequenzen flankierte Sequenz kann aus dem Genom der Zelle durch transiente Aktivierung der entsprechenden Rekombinase herausgeschnitten werden, z.B. durch
(a) Transfizieren der Zelle mit einem Vektor, umfassend eine für eine Rekombinase kodierende Nukleinsäuresequenz operativ verbunden mit einer in dieser Zelle aktiven Expressionskontrollsequenz,
(b) Kultivieren der so transfizierten Zelle unter Bedingungen, unter denen die Rekombinase exprimiert wird und aktiv ist, und
(c) gegebenenfalls Gewinnen der Zelle.

Mit dem erfindungsgemäßen Verfahren ist es nicht nur möglich ein DHFR-Gen zu inaktivieren, sondern durch eine Rekombinase vermittelte Reaktion auch Sequenzen eines DHFR-Gens, die sich zwischen den Rekombinase-Zielsequenzen befinden, sowie das eingeführte Selektionsmarkergen aus dem Genom einer Zelle herauszuschneiden.

Wenn die von Rekombinase-Zielsequenzen flankierte Sequenz ein positives Selektionsmarkergen beinhaltet, ist die diese Sequenz enthaltende Zelle antibiotikumresistent. Sie kann somit leicht nach dem Fachmann bekannten Verfahren selektioniert werden.

Ein weiterer Vorteil einer nach dem erfindungsgemäßen Verfahren hergestellten DHFR-negativen Zelle ist, daß ihre Eigenschaften durch dem Fachmann bekannte Methoden charakterisiert werden können und die Zellen anschließend für weitere Verfahren verwendet werden können. Darüber hinaus können durch die an dem DHFR-Genlocus eingeführte Rekombinase-Zielsequenz ortsspezifisch Nukleinsäuresequenzen in das Genom integriert werden.

Eine weitere bevorzugte Auführungsform betrifft ein Verfahren zum Einführen eines heterologen DHFR-Gens in eine eukaryontische Zelle, das dadurch gekennzeichnet ist, daß eine nach dem oben beschriebenen Verfahren erhaltene DHFR-negative Zelle
(a) transfiziert wird mit einem dritten Vektor, umfassend
   (i) gegebenenfalls ein positives Selektionsmarkergen, das sich vorzugsweise von dem positiven Selektionsmarkergen des ersten Vektors unterscheidet,
   (ii) eine für eine DHFR kodierende Nukleinsäuresequenz,
   (iii) eine zu amplifizierende für ein Protein kodierende Nukleinsäuresequenz in exprimierbarer Form und

   wobei die Nukleinsäuresequenz aus den Teilsequenzen (i), (ii) und (iii) 5'-seitig und 3'-seitig jeweils von mindestens einer Rekombinase-Zielsequenz flankiert ist,
(b) die transfizierte Zelle unter Bedingungen kultiviert wird, unter denen eine Integration der von Rekombinase-Zielsequenzen flankierten Nukleinsäuresequenz an der bereits im Genom der Zelle befindlichen Rekombinase-Zielsequenz erfolgt und
(c) die gemäß Schritt (b) erhaltene Zelle gewonnen wird.

Das positive Selektionsmarkergen, das DHFR-Gen und das für das gewünschte Protein kodierende Zielgen sind vorzugsweise jeweils operativ mit einer in der Zelle aktiven oder aktivierbaren Expressionskontrollsequenz verbunden. Prinzipiell ist auch ein polycistronisches Konstrukt mit internen ribosomalen Bindestellen möglich. Die zu amplifizierende Nukleinsäuresequenz des Zielgens sollte jedoch durch einen separaten Promotor getrieben sein. Besonders bevorzugte Expressionskontrollsequenzen sind virale Promotoren/Enhancer. Am meisten bevorzugt ist zur Expression des Proteins ein CMV-Promotor.

Vorteilhaft ist, daß die erfindungsgemäße Integration heterologer Sequenzen in das Genom einer Zelle ortsspezifisch erfolgt und somit Interferenzen der heterologen Sequenzen mit genomischen Sequenzen ausgeschlossen sind. Die daraus resultierenden weiter oben beschriebenen Nachteile, wie beispielsweise instabile Herstellungsklone, können so vermieden werden.

Zur Steigerung der Expressionsrate einer heterologen für ein Protein kodierenden Nukleinsäuresequenz kann ein Amplifikationsschritt mit Methotrexat nach bekannten Verfahrensschritten durchgeführt werden.

Ein weiterer Gegenstand der vorliegenden Erfindung ist ein Vektor, umfassend
(i) gegebenenfalls ein positives Selektionsmarkergen,
(ii) eine für ein DHFR kodierende Nukleinsäuresequenz, und
(iii) eine für ein gewünschtes Protein kodierende Nukleinsäuresequenz in exprimierbarer Form,

wobei die Nukleinsäuresequenz aus den Teilsequenzen (i), (ii) und (iii) 5'-seitig und 3'-seitig jeweils von mindestens einer Rekombinase-Zielsequenz flankiert ist.

Noch ein weiterer Gegenstand der vorliegenden Erfindung ist ein Vektor für die homologe Rekombination, umfassend
(i) gegebenenfalls ein positives Selektionsmarkergen,
(ii) mindestens jeweils eine Rekombinase-Zielsequenz, die die Sequenz (i) flankiert und
(iii) die Sequenzen (i) und (ii) flankierende DNA-Sequenzen, die homolog zu einer endogen in einer Zelle vorliegenden DHFR-Nukleinsäuresequenz sind, um eine homologe Rekombination zu erlauben, und
(iv) gegebenenfalls ein negatives Selektionsmarkergen außerhalb, vorzugsweise 3'-seitig der homologen Sequenzen (iii).

Weiterhin betrifft die Erfindung eine eukaryontische Zelle, vorzugsweise eine humane Zelle, erhältlich durch ein oben beschriebenes Verfahren. Diese Zelle ist dadurch gekennzeichnet, daß
(a) mindestens eine endogene, für eine DHFR kodierende Nukleinsäuresequenz inaktiviert ist, vorzugsweise beide endogenen Allele und
(b) im Bereich dieser für DHFR kodierenden Nukleinsäuresequenz mindestens eine Rekombinase-Zielsequenz in das Genom integriert ist.

Schließlich noch ein weiterer Gegenstand der Erfindung ist eine eukaryontische Zelle, vorzugsweise eine humane Zelle, die gekennzeichnet ist durch eine heterologe Nukleinsäuresequenz im Bereich eines endogenen DHFR-Genlocus umfassend
(i) eine für eine DHFR kodierende Nukleinsäuresequenz,
(ii) eine für ein gewüschtes Protein kodierende Nukleinsäuresequenz und
(iii) mindestens eine Rekombinase-Zielsequenz.

Die Erfindung wird durch die nachfolgenden Beispiele, Figuren und das Sequenzprotokoll erläutert.

### Figurenbeschreibung

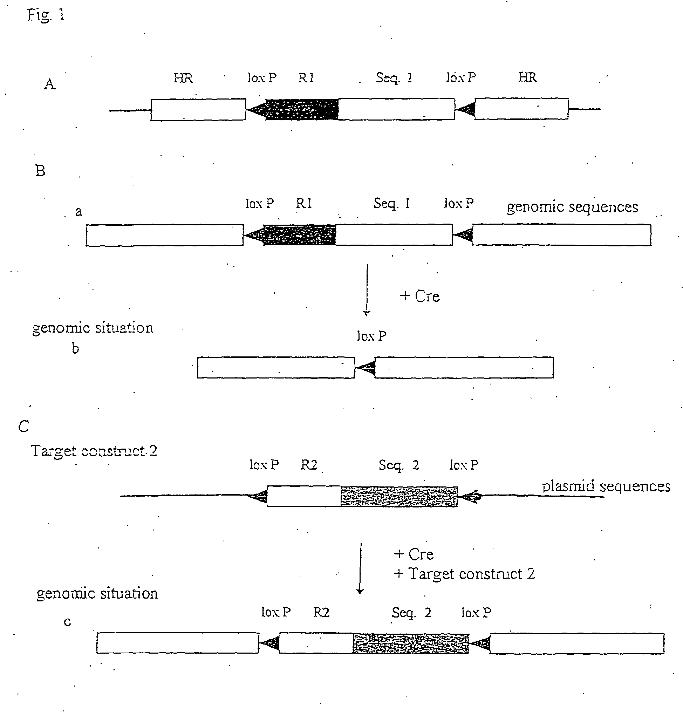

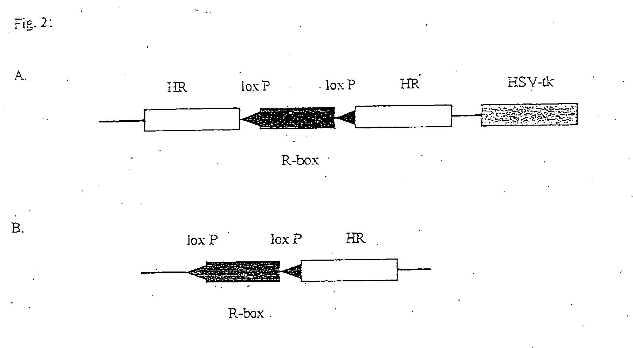

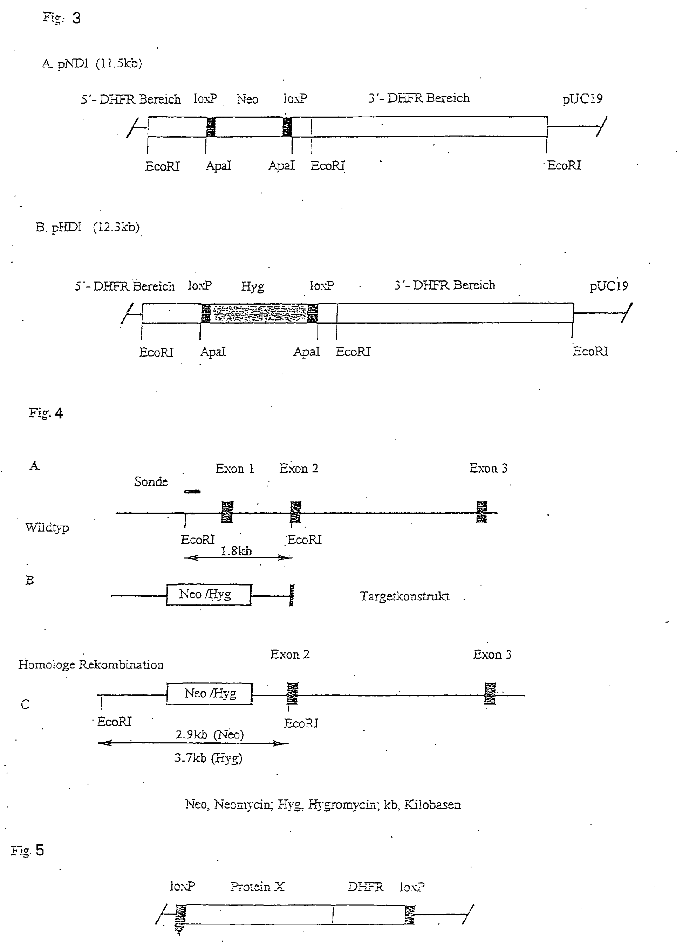

**Figur 1**
   (A) zeigt einen Vektor für die homologe Rekombination, der als erster Vektor verwendet wird. HR: homologe Sequenz, Seq 1: erste heterologe Expressionskontrollsequenz, R1: positives Selektionsmarkergen, loxP: loxP-Sequenz mit Orientierung,
   (B) zeigt genomische Sequenzen
      (a) nach erfolgter homologer Rekombination,
      (b) nach durch eine Cre-Rekombinase katalysiertem Herausschneiden einer von loxP-Sequenzen flankierten Sequenz,
   (C) zeigt einen Vektor für eine Cre-Rekombinase vermittelte Integration, der eine Sequenz zwischen loxP-Sequenzen angeordnet umfaßt,
      (c) zeigt genomische Sequenzen nach Integration eines zweiten Vektors an der loxP-Sequenz. R2: positives Selektionsmarkergen, welches sich gegebenenfalls von R1 unterscheidet, Seq 2: zweite heterologe Expressionskontrollsequenz.
**Figur 2**
   (A) zeigt einen Vektor für die homologe Rekombination HR: homologe Sequenz, R-box: positives und gegebenenfalls negatives Selektionsmarkergen, loxP: loxP-Sequenz mit Orientierung, HSV-tk: Herpes simplex-Thymidinkinase;
   (B) zeigt einen Vektor für die homologe Rekombination mit einseitiger homologer Sequenz.
**Figur 3**
   (A) zeigt den Vektor pNDI für die homologe Rekombination in einen DHFR-Genlocus. Ein positives Selektionsmarkergen (Neo) wird flankiert von zwei loxP-Sequenzen. 5'-seitig der einen loxP-Sequenz bzw. 3'-seitig der anderen loxP-Sequenz befinden sich die zu einem DHFR-Gen homologen Sequenzen (5'-, 3'-DHFR-Bereich).
   (B) zeigt den Vektor pHDI für die homologe Rekombination in einen DHFR-Genlocus. Ein positives Selektionsmarkergen (Hyg) wird flankiert von zwei loxP-Sequenzen. 5'-seitig der einen loxP-Sequenz bzw. 3'-seitig der anderen loxP-Sequenz befinden sich die zu einem DHFR-Gen homologen Sequenzen (5'-, 3'-DHFR-Bereich).
**Figur 4**
   (A) zeigt den genomischen Aufbau eines DHFR-Gens mit Exon 1, Exon 2 und Exon 3, sowie den dazwischen liegenden Introns,
   (B) zeigt schematisch einen Figur 3 entsprechenden Vektor, Targetkonstrukt,
   (C) zeigt die genomische Struktur nach erfolgter homologer Rekombination des Vektors für die homologe Rekombination in ein DHFR-Gen. Der Abstand zwischen den EcoRI-Schnittstellen beträgt bei Verwendung des Vektors pNDI 2,9 kb bzw. 3,7 kb bei Verwendung des Vektors pHDI. Neo: Neomycin, Hyg: Hygromycin, kb: Kilobasen
**Figur 5**
   zeigt einen Vektor, der eine für ein Protein X kodierende Nukleinsäuresequenz und eine für ein DHFR-Protein kodierende Nukleinsäuresequenz jeweils einschließlich regulatorischer Sequenzen umfaßt, die von zwei loxP-Sequenzen flankiert sind. Dieser Vektor kann zur Cre-Rekombinase katalysierten Integration in das Genom in eine loxP-Sequenz verwendet werden.

### SEQ ID NO. 1 zeigt eine IoxP-Sequenz.

### BEISPIELE

### Beispiel 1 Herstellung von DHFR-negativen Zellen

In einem ersten Schritt werden erfindungsgemäße Vektoren für die Rekombination hergestellt. Diese Vektoren werden in einem zweiten Schritt in humane Zellinien transfiziert und auf homologe Rekombinationsereignisse gescreent. Auf diese Weise kann erst ein, dann das 2. Allel für das DHFR-Gen inaktiviert werden.

### DHFR-Vektor für die homologe Rekombination

Das humane DHFR-Gen ist auf Chromosom 5 lokalisiert und umfaßt 30 kb, die sich in 6 Exons gliedern. Ein 1,8 kb großes EcoR1-Fragment, welches Teile des Promotors, Teile von Exon 2 und das komplette Exon 1 enthält, wird zur Herstellung des Vektors für die homologe Rekombination verwendet. Exon 1 wird durch einen Aapl-Verdau entfernt und in die entstandene Lücke (0,45 kb) wird das Neo-(1,4 kb)- bzw. Hyg-(2,2 kb)-Resistenzgen über Linker eingesetzt. Diese Linker enthalten zusätzlich zu den Adaptornukleotiden die minimale Sequenz TAT TG AAG CAT ATT ACA TAC GAT ATG CTT CAA TA (loxP-Sequenz). Die Linkersequenzen sind in der gleichen Orientierung, das Resistenzgen bevorzugt antisense zum DHFR-Gen angeordnet. Nachdem das Resistenzgen eingesetzt wurde, wird die Homologieregion vergrößert. Hierzu wird der Vektor um das EcoRI Fragmente aus dem 3'-Bereich (6,0 kb) erweitert (Fig. 3). Man erhält somit die erfindungsgemäßen Targetkonstrukte pNDI (11,5 kb) und pHDI (12,3 kb) .

Nach erfolgreicher homologer Rekombination sind das komplette Exon 1 (Aminosäuren 1-28) und Teile des Promotors des DHFR-Gens entfernt. Die Zelle kann nun kein funktionelles DHFR-Protein mehr exprimieren.

### Transfektion von Zellen

Die verwendeten humanen Zellinien sollten nicht polyploid für Chromosom 5 sein und nicht unter MTX Selektion gehalten worden sein. In beiden Fällen würden mehr als 2 Allele zu inaktivieren sein.

### HeLa 53-Zellen (ATCC CCL-2.2)

Die Zellen werden in Gewebekulturflaschen in RPMI 1640 Medium, 10% fötalem Kälberserum, 2 mM L-Glutamin und 1 mM MEM (nichtessentielle Aminosäuren) kultiviert. Die Inkubation erfolgt bei 37°C und 5% CO₂. Der Elektroporationspuffer enthält 20 mM Hepes, 138 mM NaCl, 5 mM KCI, 0,7 mM Na₂HPO₄. 6 mM D-Glucose-Monohydrat, pH 7,0. 10 µg linearisierte Vektor-DNA (pNDI) wird bei 960 *µ*F und 250V in 1 x 10⁷ Zellen elektroporiert (Biorad Gene Pulser). Nach der Elektroporation werden die Zellen in Medium mit 600 *µ*g/ml G418 (Geneticin Boehringer Mannheim) aufgenommen und kultiviert. Nach 10 Tagen Selektion (Mediumwechsel alle 2 Tage) werden die positiven Klone isoliert und expandiert.

### HT1080 Zellen (ATCC CCL-121.)

Die Kultivierung und Selektion der Zellen erfolgt wie für HeLa S3-Zellen beschrieben mit DMEM-Medium mit 10 % fötalem Kälberserum, 2 mM L-Glutamin und 1 mM Natriumpyruvat.

### Namalwazellen (ATCC CRL-1432)

Diese Zellinie ist eine Suspensionszellinie und muß entsprechend kultiviert werden. Das Medium entspricht dem für HeLa S3-Zellen beschriebenen. Nach der Transfektion werden die Zellen auf vierzig 96well-Platten verteilt. Positive Klone werden in 48-, 24-, 12- und 6-well Platten expaniert. Die Selektion erfolgt mit 1 mg/ml G418.

### Nachweis der DHFR-negativen (+/-) Zellen

Der Nachweis der Insertion des Vektors wird mittels Southern Blot Analyse oder PCR durchgeführt. Bei korrekt erfolgter homologer Rekombination wird nach EcoR1-Verdau zusätzlich zu einer 1,8 kb Bande, die das intakte DHFR-Gen repräsentiert, eine 2,9 kb Bande nachgewiesen, welche durch die Insertion des Neo-Gens entstanden ist (Figur 4c). Mischklone (ungleiches Verhältnis der Bandenintensität in Southern Blot) werden über Einzelzellablage im FACS getrennt, subkloniert und anschließend expandiert. In den als positiv identifizierten Klonen ist ein Allel des DHFR-Gen inaktiviert.

### Erzeugung von DHFR-negativen (-/-) Zellen

Zellklone, in denen ein DHFR-Allel (+ /-) inaktiviert ist, können einer erneuten homologen Rekombination unterzogen werden. Hierzu werden sie wie oben beschrieben mit 10 µg linearisierter DNA des Vektors pHDI transfiziert. Die Selektion erfolgt in Medium mit 500 *µ*g/ml Hygromycin B (Boehringer Mannheim).

Durch eine Erhöhung der G418-Konzentration im Medium kann der Selektionsdruck auf DHFR^{+/-} Zellen erhöht werden und DHFR^{-/-} Zellen erhalten werden. Eine genetische Konversion führt zu einer interchromosomalen Rekombination, wodurch das zweite DHFR-Allel inaktiviert wird.

Die DHFR^{-/-} Zellen enthalten zwei inaktivierte DHFR-Allele und können kein Tetrahydrofolat mehr synthetisieren. Daher muß dem Medium Thymidin, Glycin und Purin zugegeben werden (Supplementation). Gegebenenfalls werden die Zellen in α⁻ Medium (Gibco BRL) kultiviert.

Der Nachweis der DHFR^{-/-} Zellen erfolgt wie oben beschrieben. Bei homozygoten DHFR-negativen Zellen ist keine Wildtypbande (1,8 kb) nachweisbar. Zellen, die mit pHDI transfiziert wurden, zeigen nach homologer Rekombination eine neue 3,7 kb Bande in EcoRI Southern Blot (Figur 4c).

### Verwendung von DHFR-negativen Zellen (-/-)

Die erfindungsgemäßen Zellen können zur Hochproduktion von Proteinen verwendet werden. Hierzu wird ein erfindüngsgemäßer Vektor (gemäß Figur 5) und ein eine Cre-Rekombinase kodierender Expressionsvektor in die DHFR^{-/-} Zellen transfiziert. Die Cre-Rekombinase entfernt die Antibiotikum-Resistenz aus dem DHFR-Genlocus und integriert in die loxP-Sequenz im Genom der DHFR^{-/-} Zelle den erfindungsgemäßen Vektor. Die Zellen werden wieder antibiotikumsensitiv und unabhängig von einer Thymidin, Glycin und Purin Supplementation.

Die Selektion kann durch Verwendung eines Mediums ohne Supplementation oder durch Zugabe eines geeigneten Antibiotikums zum Kulturmedium erfolgen. Das Antibiotikum entspricht dabei dem Resistenzgen, das durch die Cre-Rekombinase aus dem Genom der Zelle entfernt wurde. Enthält der in die loxP-Sequenz integrierte Vektor ein positives Selektionsmarkergen, kann die Selektion durch Zugabe dieses Antibiotikums zum Medium durchgeführt werden.

### Erhöhung der Produktionsleistung durch Genamplifikation

Um die Produktionsleistung der Zellen für das rekombinante Protein zu erhöhen, wird eine Methotrexat (MTX)-Selektion durchgeführt, wodurch das in die Zelle eingeführte DHFR-Gen und die heterologe für ein Protein kodierende Nukleinsäuresequenz amplifiziert werden.

Um eine Amplifikation zu erreichen, werden die Zellen in Gegenwart von steigenden Konzentrationen (100-1000 mM) MTX kultiviert. Der Grad der Amplifikation erfolgt über densitometrische Auswertung von vergleichenden Southern Blots (vor, während, nach MTX-Zugabe).

Die nach dem Amplifikationsschritt erhaltenen erfindungsgemäßen Zellen enthalten an dem loxP-Locus viele Kopien des eingeführten DHFR-Gens und der eingeführten heterologen Nukleinsäuresequenz. Sie zeichnen sich durch eine hohe Produktionsleistung der heterologen Nukleinsäure aus.

Im Folgenden sind bevorzugte Ausführungsformen der Erfindung als Bestandteil der Beschreibung dargestellt:
1. Verfahren zur Bereitstellung einer DHFR-negativen eukaryontischen Zelle,
   **dadurch gekennzeichnet,**
   daß
   (a) die Zelle transfiziert wird mit einem ersten Vektor, umfassend
      (i) mindestens eine Zielsequenz für eine ortsspezifische Rekombinase,
      (ii) die Sequenz (i) flankierende DNA-Sequenzen, die homolog zu einer endogen in der Zelle vorliegenden DHFR-Nukleinsäuresequenz sind, um eine homologe Rekombination zu erlauben, und
      (iii) gegebenenfalls ein positives und gegebenenfalls ein negatives Selektionsmarkergen,
   (b) die transfizierte Zelle unter Bedingungen kultiviert wird, unter denen eine homologe Rekombination des Vektors erfolgt, und
   (c) die gemäß Schritt (b) erhaltene Zelle gewonnen wird.
2. Verfahren nach Punkt 1,
   **dadurch gekennzeichnet,**
   daß man als Rekombinase-Zielsequenzen loxP-Sequenzen verwendet.
3. Verfahren nach einem der Punkte 1 oder 2,
   **dadurch gekennzeichnet,**
   daß die für das positive Selektionsmarkergen kodierende Nukleinsäuresequenz ein Neomycin-, Kanamycin-, Geneticin- oder Hygromycin-Resistenzgen ist.
4. Verfahren nach einem der Punkte 1 bis 3,
   **dadurch gekennzeichnet,**
   daß die für das negative Selektionsmarkergen kodierende Nukleinsäuresequenz ein Thymidin-Kinase-Gen (TK) oder/und Hypoxanthin-Guanin-Phosphoribosyltransferase-Gen (HGPRT) ist.
5. Verfahren nach einem der Punkte 1 bis 4,
   **dadurch gekennzeichnet,**
   daß die von den Rekombinase-Zielsequenzen flankierte Sequenz aus dem Genom der Zelle durch eine transiente Aktivierung der entsprechenden Rekombinase herausgeschnitten wird.
6. Verfahren zum Einführen eines heterologen DHFR-Gens in eine eukaryontische Zelle,
   **dadurch gekennzeichnet,**
   daß eine durch das Verfahren nach Punkt 5 erhaltene Zelle
   (a) transfiziert wird mit einem dritten Vektor, umfassend
      (i) gegebenenfalls ein positives Selektionsmarkergen, das sich vorzugsweise von dem positiven Selektionsmarkergen des ersten Vektors unterscheidet,
      (ii) eine für eine DHFR kodierende Nukleinsäuresequenz,
      (iii) eine zu amplifizierende für ein Protein kodierende Nukleinsäuresequenz und

      wobei die Nukleinsäuresequenz aus den Teilsequenzen (i), (ii) und (iii) 5'-seitig und 3'-seitig jeweils von mindestens einer Rekombinase-Zielsequenz flankiert ist,
   (b) die transfizierte Zelle unter Bedingungen kultiviert wird, unter denen eine Integration der von Rekombinase-Zielsequenzen flankierten Nukleinsäuresequenz an der bereits im Genom der Zelle befindlichen Rekombinaase-Zielsequenz erfolgt und
   (c) die gemäß Schritt (b) erhaltene Zelle gewonnen wird.
7. Vektor, umfassend
   (i) gegebenenfalls ein positives Selektionsmarkergen,
   (ii) eine für eine DHFR kodierende Nukleinsäuresequenz und
   (iii) eine für ein gewünschtes Protein kodierende Nukleinsäuresequenz in exprimierbarer Form,

   wobei die Nukleinsäuresequenz aus den Teilsequenzen (i), (ii) und (iii) 5'-seitig und 3'-seitig jeweils von mindestens einer Rekombinase-Zielsequenz flankiert ist.
8. Vektor für die homologe Rekombination, umfassend
   (i) gegebenenfalls ein positives Selektionsmarkergen,
   (ii) mindestens jeweils eine Rekombinase-Zielsequenz, die die Sequenz (i) flankiert,
   (iii) die Sequenzen (i) und (ii) flankierende DNA-Sequenzen, die homolog zu einer endogen in einer Zelle vorliegenden DHFR-Nukleinsäuresequenz sind, um eine homologe Rekombination zu erlauben und
   (iv) gegebenenfalls ein negatives Selektionsmarkergen außerhalb der homologen Sequenzen (iii).
9. Eukaryontische Zelle, vorzugsweise humane Zelle, erhältlich durch ein Verfahren nach einem der Punkte 1 bis 6.
10. Eukaryontische Zelle, vorzugsweise humane Zelle,
   **dadurch gekennzeichnet,**
   **daß**
   (a) mindestens eine endogene, für eine DHFR kodierende Nukleinsäuresequenz inaktiviert ist und
   (b) im Bereich dieser für DHFR kodierenden Nukleinsäuresequenz mindestens eine Rekombinase-Zielsequenz in das Genom integriert ist.
11. Eukaryontische Zelle, vorzugsweise humane Zelle,
   **gekennzeichnet durch**
   eine heterologe Nukleinsäuresequenz im Bereich eines endogenen DHFR-Genlocus, umfassend
   (i) eine für eine DHFR kodierende Nukleinsäuresequenz,
   (ii) eine für ein gewünschtes Protein kodierende Nukleinsäuresequenz und
   (iii) mindestens eine Rekombinase-Zielsequenz.

## Patentansprüche

1. Verfahren zur Bereitstellung einer DHFR-negativen eukaryontischen Zelle,
**dadurch gekennzeichnet,**
**dass**
(a) die Zelle transfiziert wird mit einem ersten Vektor, umfassend
(i) mindestens eine Zielsequenz für eine ortsspezifische Rekombinase,
(ii) die Sequenz (i) flankierende DNA-Sequenzen, die homolog zu einer endogen in der Zelle vorliegenden DHFR-Nukleinsäuresequenz sind, um eine homologe Rekombination zu erlauben, und
(iii) gegebenenfalls ein positives und gegebenenfalls ein negatives Selektionsmarkergen,
(b) die transfizierte Zelle unter Bedingungen kultiviert wird, unter denen eine homologe Rekombination des Vektors erfolgt, und
(c) die gemäß Schritt (b) erhaltene Zelle gewonnen wird.

2. Verfahren zum Einführen eines heterologen DHFR-Gens in eine eukaryontische Zelle,
**dadurch gekennzeichnet,**
**dass** eine durch das Verfahren nach Anspruch 1 erhaltene Zelle
(a) transfiziert wird mit einem dritten Vektor, umfassend
(i) gegebenenfalls ein positives Selektionsmarkergen, das sich vorzugsweise von dem positiven Selektionsmarkergen des ersten Vektors unterscheidet,
(ii) eine für eine DHFR kodierende Nukleinsäuresequenz,
(iii) eine zu amplifizierende für ein Protein kodierende Nukleinsäuresequenz in exprimierbarer Form und
wobei die Nukleinsäuresequenz aus den Teilsequenzen (i), (ii) und (iii) 5'-seitig und 3'-seitig jeweils von mindestens einer Rekombinase-Zielsequenz flankiert ist,
(b) die transfizierte Zelle unter Bedingungen kultiviert wird, unter denen eine Integration der von Rekombinase-Zielsequenzen flankierten Nukleinsäuresequenz an der bereits im Genom der Zelle befindlichen Rekombinaase-Zielsequenz erfolgt und
(c) die gemäß Schritt (b) erhaltene Zelle gewonnen wird.

3. Vektor, umfassend
(i) gegebenenfalls ein positives Selektionsmarkergen,
(ii) eine für eine DHFR kodierende Nukleinsäuresequenz und
(iii) eine für ein gewünschtes Protein kodierende Nukleinsäuresequenz in exprimierbarer Form,
wobei die Nukleinsäuresequenz aus den Teilsequenzen (i), (ii) und (iii) 5'-seitig und 3'-seitig jeweils von mindestens einer Rekombinase-Zielsequenz flankiert ist.

4. Vektor für die homologe Rekombination, umfassend
(i) gegebenenfalls ein positives Selektionsmarkergen,
(ii) mindestens jeweils eine Rekombinase-Zielsequenz, die die Sequenz (i) flankiert,
(iii) die Sequenzen (i) und (ii) flankierende DNA-Sequenzen, die homolog zu einer endogen in einer Zelle vorliegenden DHFR-Nukleinsäuresequenz sind, um eine homologe Rekombination zu erlauben und
(iv) gegebenenfalls ein negatives Selektionsmarkergen außerhalb der homologen Sequenzen (iii).

5. Eukaryontische Zelle, vorzugsweise humane Zelle,
**dadurch gekennzeichnet,**
**dass**
(a) mindestens eine endogene, für eine DHFR kodierende Nukleinsäuresequenz inaktiviert ist und
(b) im Bereich dieser für DHFR kodierenden Nukleinsäuresequenz mindestens eine Rekombinase-Zielsequenz in das Genom integriert ist.

6. Eukaryontische Zelle, vorzugsweise humane Zelle,
**gekennzeichnet durch**
eine heterologe Nukleinsäuresequenz im Bereich eines endogenen DHFR-Genlocus, umfassend
(i) eine für eine DHFR kodierende Nukleinsäuresequenz,
(ii) eine für ein gewünschtes Protein kodierende Nukleinsäuresequenz und
(iii) mindestens eine Rekombinase-Zielsequewz.

## Revendications

1. Procédé de préparation d'une cellule eucaryote négative pour la DHFR, **caractérisé en ce que**
(a) on transfecte la cellule avec un premier vecteur comprenant
(i) au moins une séquence cible pour une recombinase spécifique d'un site,
(ii) des séquences d'ADN flanquant la séquence (i), qui sont homologues à une séquence d'acide nucléique de DHFR se trouvant de manière endogène dans la cellule pour permettre une recombinaison homologue, et
(iii) éventuellement un gène de marqueur de sélection positive et éventuellement un gène de marqueur de sélection négative,
(b) on cultive la cellule transfectée dans des conditions dans lesquelles il se produit une recombinaison homologne du vecteur, et
(c) on récupère la cellule obtenue selon l'étape (b).

2. Procédé d'introduction d'un gène de DHFR hétérologue dans une cellule eucaryote, **caractérisé en ce que**
(a) on transfecte une cellule obtenue par le procédé selon la revendication 1 avec un troisième vecteur, comprenant
(i) éventuellement un gène de marqueur de sélection positive qui se distingue de préférence du gène de marqueur de sélection positive du premier vecteur,
(ii) une séquence d'acide nucléique codant pour une DHFR,
(iii) une séquence d'acide nucléique codant pour une protéine, à amplifier, sous une forme exprimable, et
la séquence d'acide nucléique constituée des séquences partielles (i), (ii) et (iii) étant flanquée du côté 5' et du côté 3' par au moins une séquence cible pour une recombinase,
(b) on cultive la cellule transfectée dans des conditions dans lesquelles il se produit une intégration de la séquence d'acide nucléique flanquée des séquences cibles pour une recombinase au niveau de la séquence cible pour une recombinase se trouvant déjà dans le génome de la cellule, et
(c) on récupère la cellule obtenue selon l'étape (b).

3. Vecteur comprenant
(i) éventuellement un gène de marqueur de sélection positive,
(ii) une séquence d'acide nucléique codant pour une DHFR et
(iii) une séquence d'acide nucléique sous une forme exprimable codant pour une protéine désirée;
la séquence d'acide nucléique constituée des séquences partielles (i), (ii) et (iii) étant flanquée du côté 5' et du côté 3' à chaque fois par au moins une séquence cible pour une recombinase.

4. Vecteur pour la recombinaison homologue, comprenant
(i) éventuellement un gène de marqueur de sélection positive,
(ii) au moins à chaque fois une séquence cible pour une recombinase qui flanque la séquence (i),
(iii) des séquences d'ADN flanquant les séquences (i) et (ii), qui sont homologues à une séquence d'acide nucléique de DHFR présente de façon endogène dans une cellule, pour permettre une recombinaison homologue, et
(iv) éventuellement un gène de marqueur de sélection négative en-dehors des séquences homologues (iii).

5. Cellule eucaryote, de préférence cellule humaine, **caractérisée en ce que**
(a) au moins une séquence d'acide nucléique codant pour une DHFR est inactivée et
(b) dans la région de cette séquence d'acide nucléique codant pour une DHFR, au moins une séquence cible pour une recombinase est intégrée dans le génome.

6. Cellule eucaryote, de préférence cellule humaine, **caractérisée par** une séquence d'acide nucléique hétérologue dans la région d'un locus de gène de DHFR endogène, comprenant
(i) une séquence d'acide nucléique codant pour une DHFR,
(ii) une séquence d'acide nucléique codant pour une protéine désirée, et
(iii) au moins une séquence cible pour une recombinase.

## Claims

1. Process for providing a DHFR-negative eukaryotic cell,
**characterized in that**
(a) the cell is transfected with a first vector comprising
(i) at least one target sequence for a site-specific recombinase,
(ii) DNA sequences flanking sequence (i) which are homologous to a DHFR nucleic acid sequence that is present endogenously in the cell in order to allow a homologous recombination and
(iii) optionally a positive selection marker gene and optionally a negative selection marker gene,
(b) the transfected cell is cultured under conditions under which a homologous recombination of the vector takes place and
(c) the cell obtained according to step (b) is isolated.

2. Process for introducing a heterologous DHFR gene into a eukaryotic cell,
**characterized in that**
a cell obtained by the process according to item 1
(a) is transfected with a third vector comprising
(i) optionally a positive selection marker gene which preferably differs from the positive selection marker gene of the first vector,
(ii) a nucleic acid sequence coding for a DHFR,
(iii) a nucleic acid sequence to be amplified coding for a protein in which each nucleic acid sequences from the partial sequences (i), (ii) and (iii) is flanked on the 5' side and 3' side by at least one recombinase target sequence,
(b) the transfected cell is cultured under conditions under which the nucleic acid sequence flanked by recombinase target sequences is integrated into the recombinase target sequence that is already present in the genome of the cell and
(c) the cell obtained according to step (b) is isolated.

3. Vector, comprising
(i) optionally a positive selection marker gene,
(ii) a nucleic acid sequence coding for a DHFR and
(iii) a nucleic acid sequence in an expressible form coding for a desired protein
in which each nucleic acid sequence from the partial sequences (i), (ii) and (iii) is flanked on the 5' side and 3' side by at least one recombinase target sequence.

4. Vector for homologous recombination comprising,
(i) optionally a positive selection marker gene
(ii) at least one recombinase target sequence in each case which flanks the sequence (i),
(iii) DNA sequences flanking the sequences (i) and (ii) which are homologous to a DHFR nucleic acid sequence that is present endogenously in a cell in order to allow a homologous recombination and
(iv) optionally a negative selection marker gene outside the homologous sequences (iii).

5. Eukaryotic cell, preferably a human cell,
**characterized in that**
(a) at least one endogenous nucleic acid sequence coding for a DHFR is inactivated and
(b) at least one recombinase target sequence is integrated into the genome in the region of this nucleic acid sequence coding for DHFR

6. Eukaryotic cell, preferably a human cell,
**characterized by**
a heterologous nucleic acid sequence in the region of an endogenous DHFR gene locus, comprising
(i) a nucleic acid sequence coding for DHFR,
(ii) a nucleic acid sequence coding for a desired protein and
(iii) at least one recombinase target sequence.


### SEQUENZPROTOKOLL

(1) ALLGEMEINE ANGABEN:
   (i) ANMELDER:
      (A) NAME: Boehringer Mannheim GmbH
      (B) STRASSE: Sandhofer strasse 112-132
      (C) ORT: Mannheim
      (E) LAND: Deutschland
      (F) POSTLEITZAHL: D-68305
   (ii) BEZEICHNUNG DER ERFINDUNG: Optimierung von Zellen fuer die endogene Genaktivierung
   (iii) ANZAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1
   (i) SEQUENZKENNZEICHEN:
      (A) LÄNGE: 34 Basenpaare
      (B) ART: Nucleotid
      (C) STRANGFORM: beides
      (D) TOPOLOGIE: linear
   (xi) SEQUENZBESCHREIBUNG: SEQ ID NO: 1
   TATTGAAGCA TATTACATAC GATATGCTTC AATA 34